# EUROPEAN PATENT SPECIFICATION

(11) **EP 3 190 413 B1**
(45) Date of publication and mention of the grant of the patent: **11.08.2021**
(21) Application number: 15844112.1
(22) Date of filing: 24.09.2015
(51) Int. Cl.: G01N 33/50, A61K 45/00, A61P 25/24, A61K 31/135, A61K 31/343, A61K 31/381, A61K 31/4525, A61K 31/165

(54) **METHOD FOR PREDICTING DEPRESSION TREATMENT DRUG ALTERNATIVES**
VERFAHREN ZUR VORHERSAGE VON ARZNEIMITTELALTERNATIVEN FÜR DIE BEHANDLUNG VON DEPRESSION
MÉTHODE DE PRÉDICTION D'ALTERNATIVES AUX MÉDICAMENTS DE TRAITEMENT DE LA DÉPRESSION

(30) Priority: 26.09.2014 JP 2014196043
(43) Date of publication of application: 12.07.2017
(73) Proprietor: Human Metabolome Technologies, Inc., Tsuruoka-shi, Yamagata 997-0052 (JP); Gyokikai Medical Corporation, Tokyo 107-0062 (JP)
(72) Inventor: KAWAMURA, Noriyuki, Tokyo 107-0062 (JP); SATO, Hajime, Tsuruoka-shi Yamagata 997-0052 (JP); YAMAKI, Kumi, Tsuruoka-shi Yamagata 997-0052 (JP); FUJIMORI, Tamaki, Tokyo 160-0022 (JP); YAMAMOTO, Hiroyuki, Tsuruoka-shi Yamagata 997-0052 (JP); OHASHI, Yoshiaki, Tsuruoka-shi Yamagata 997-0052 (JP)
(74) Representative: Gallego Jiménez, José Fernando
(86) International application number: PCT/JP2015/076903
(87) International publication number: WO 2016/047677

(56) References cited:
- EP-A1- 2 778 233
- WO-A1-2011/019072
- JP-A- 2009 007 278
- JP-A- 2014 101 324
- US-A1- 2012 282 592
- SCHACHT ALEXANDER ET AL: "Depression symptom clusters and their predictive value for treatment outcomes: Results from an individual patient data meta-analysis of duloxetine trials", JOURNAL OF PSYCHIATRIC RESEARCH, vol. 53, 1 June 2014 (2014-06-01), pages 54-61, XP028839389, ISSN: 0022-3956, DOI: 10.1016/J.JPSYCHIRES.2014.02.001
- None

## Description

### Technical Field

The present invention relates to a method for predicting options of depression treatment drugs, more specifically to a method for objectively and easily predicting options of depression treatment drugs.

### Background Art

Diagnosis of psychiatric diseases such as depression is carried out on the basis of International Statistical Classification of Diseases and Related Health Problems (ICD) by World Health Organization, and Diagnostic and Statistical Manual of Mental Disorders (DSM) published by American Psychiatric Association.

ICD is a classification announced by World Health Organization as an international statistical standard for causes of death and diseases. It is utilized for international comparison of information on statistics or the like of causes of death and diseases, managements of medical records in medical institutions, and the like.

DSM presents a common language and a standard criterion for classification of psychiatric disorders. The diagnostic criterion of DSM-IV-TR for major depressive disorders is that 5 or more of the following 9 symptoms: (1) depressed mood; (2) remarkable decline of interest/pleasure; (3) fatigue/decline of energy; (4) anorexia/appetite excessive; (5) insomnia/plethoric sleep; (6) feelings of worthlessness/guilt; (7) decline of concentration/ability to think; (8) psychomotor irritation/psychomotor retardation; (9) suicidal ideation/suicidal attempt are exhibited within 2 weeks, and at least (1) or (2) are exhibited among these symptoms. In relation to the symptoms of (1) and (2), among the patients with major depressive disorder, some patients exhibit only (1), some patients exhibit only (2), and some patients exhibit both (1) and (2), and thus they are considered to be a population of hetero patients.

The diagnostic criterion of DSM-IV-TR for depressive disorders is clearer than that of DSM-III in order to solve the incongruity in diagnosis. However, the diagnostic criteria have problems that they include no biological indicator, that they are not directly linked to treatments, that they are extendedly interpreted and increase patients unnecessarily, and the like. Although DSM is useful for epidemiological investigation, it was not made for the purpose of precisely diagnosing individual patient.

The hypothesis is convincing that the biological cause of depression is depletion of three neurotransmitters, i.e., serotonin, noradrenaline and dopamine. It is generally believed among experts that depression is an aggregate of different diseases. Diagnosis of psychiatric disease is considered to have many errors because subjectivity is involved.

In order to establish a definition of psychiatric disease, biomarkers with objective and biological indicators are required. The inventors previously found that phosphoethanolamine in blood could be a biomarker of depression (Patent Document 1). Specifically, if the blood phosphoethanolamine level of a subject is lower than the blood phosphoethanolamine levels in healthy persons, the subject is determined to suffer from depression. Depressions exhibiting decreasing phosphoethanolamine level as an indicator can be said to be a highly homogeneous population characterized by biological indicators. Diagnosing depression with a low phosphoethanolamine level can give an objective definition for a psychiatric disease.

On the basis of their functions, the depression treatment drugs are roughly classified into: serotonin/noradrenaline reuptake inhibitors (SNRI); selective serotonin reuptake inhibitors (SSRI); noradrenergic/serotoninergic antidepressants (NaSSA); tricyclic antidepressants; and tetracyclic antidepressants.

The SNRI is a drug that improves depressive symptoms by inhibiting the reabsorption of serotonin and noradrenaline in a synapse. Serotonin in a synaptic cleft is re-uptaken by a serotonin transporter. Noradrenaline is re-uptaken by a noradrenaline transporter. The SNRI acts on the serotonin transporter/noradrenaline transporter in order to inhibit the reuptake of serotonin/noradrenaline in the synaptic cleft and maintain the serotonin/noradrenaline concentration in the synaptic cleft high to some extent. As a result, effects of stimulating excitatory nerves and improving motivation and mood are exerted.

The SSRI is a drug that improves anxiety by inhibiting the reabsorption of serotonin in a synapse. Serotonin released from a synaptic neuron to a synaptic cleft acts on a serotonin receptor. Serotonin accumulated in the synaptic cleft is re-uptaken by the serotonin transporter. There is a monoamine hypothesis that a depressive symptom causes a condition in which the concentration of serotonin in the synaptic cleft is decreased and serotonin hardly acts on the serotonin receptor. The SSRI selectively acts on the serotonin transporter which reuptakes serotonin in order to inhibit reuptake of serotonin and maintain the serotonin concentration in the synaptic cleft high to some extent. The SSRI exerts an effect of calming down particularly anxiety and fear.

The NaSSA has an action of binding, as an antagonist, to a serotonin receptor having no anxiety-improving function in order to facilitate binding of serotonin to a receptor having an anxiety-improving function. In addition, the NaSSA binds, as an antagonist, to a noradrenaline receptor that suppresses noradrenaline release in order to inhibit the suppression of the noradrenaline release and maintain the serotonin/noradrenaline concentration in the synaptic cleft high to some extent.

The tricyclic antidepressant has an action of inhibiting reuptake of serotonin/noradrenaline in order to maintain the serotonin/noradrenaline concentration in the synaptic cleft high to some extent.

The tetracyclic antidepressant selectively inhibits reuptake of noradrenaline and does not inhibit reuptake of serotonin. It maintains the noradrenaline concentration in the synaptic cleft high to some extent.

### Prior Art Documents

### Patent Documents

[Patent Document 1] WO 2011019072 (BIOMARKER FOR DEPRESSION, METHOD FOR MEASURING A BIOMARKER FOR DEPRESSION, COMPUTER PROGRAM, AND RECORDING MEDIUM) . This document discloses several substances which are useful as biomarkers of depression.

### Non-patent Documents

[Non-patent Document 1] Schacht Alexander et al: "Depression symptom clusters and their predictive value for treatment outcomes: Results from an individual patient data meta-analysis of duloxetine trials", J. Psychiatr. Res., 2014, 53, 54-61. This document reviews the data of several clinical studies in major depressive disorder patients treated with anti-depressive drugs for identifying clusters of patients with similar symptom patterns at baseline in order to predict the treatment outcome for the different identified clusters.

### Summary of Invention

### Problem to be solved

Even though Patent Document 1 enables to objectively diagnose depression, prescriptions of depression treatment drugs for depressed patients are not different from the conventional prescriptions. A prescription drug is decided according to symptoms of depression and a subjective view of a doctor who performs a medical examination, and if one drug has no effect in testing it, its prescription dosage is increased, or another drug is tested. Such a method prolongs disease duration of the patient. In addition, long-term administration of an ineffective drug also causes problems of side effects such as nausea and constipation, and of increased economic burden of the patient.

Thus, an object of the present invention is to provide a method in which a phosphoethanolamine discovered as a biomarker of depression is directly linked to treatment.

### Solution to Problem

When the inventors investigated a relationship between the blood phosphoethanolamine level (hereinafter, called "blood PEA level") and administration of antidepressants, they found that the blood PEA level was decreased by administering SSRI to a depressed patient with a decreased blood PEA level, and the blood PEA level was increased by administering the SNRI thereto. On the basis of this finding, the inventors earnestly examined the above-described problems and completed the present invention.

The present invention provides a method for predicting options of depression treatment drugs, comprising the following steps:
(1) measuring a phosphoethanolamine level in blood collected from a patient suspected of depression; and
(2) measuring whether the blood phosphoethanolamine level of the patient is lower than 1.5 µM or not;
wherein if the measured value of the patient is lower than 1.5 µM, the patient suffers from depression exhibiting a decreased phosphoethanolamine level as an indicator and a serotonin/noradrenaline reuptake inhibitor is selected as depression treatment drug for the patient.

The present invention also provides for a method for predicting effects of depression treatment drugs, comprising the following steps:
(1) measuring a phosphoethanolamine level in blood collected from a depressed patient; and
(2) measuring whether the blood phosphoethanolamine level in the patient is lower than 1.5 µM or not;
wherein if the measured value of the patient is lower than 1.5 µM, the patient suffers from depression exhibiting a decreased phosphoethanolamine level as an indicator and a serotonin/noradrenaline reuptake inhibitor is predicted to be effective as a depression treatment drug.

The present invention also provides for a method for evaluating a prognosis of treatment with a depression treatment drug which is a serotonin/noradrenaline reuptake inhibitor, comprising the following steps:
(1) measuring a phosphoethanolamine level in blood collected from a depressed patient before administration of the therapeutic drug;
(2) measuring whether the phosphoethanolamine level of the patient is lower than 1.5 µM or not, wherein if the measured value of the patient is lower than 1.5 µM, the patient suffers from depression exhibiting a decreased phosphoethanolamine level as an indicator;
(3) measuring the phosphoethanolamine level in the blood collected from the depressed patient for whom the decreased blood phosphoethanolamine level was observed in the step (2) and to whom the depression treatment drug was administered; and
(4) evaluating the serotonin/noradrenaline reuptake inhibitor as effective on the basis of the fact that the blood phosphoethanolamine level in the patient after administration of the therapeutic drug was higher than 1.5 µM.

In a preferred embodiment, in the step (4), the prognosis of the patient is evaluated as partial depression remission or depression remission on the basis of the increase degree of the blood phosphoethanolamine level in the patient after administration of the therapeutic drug compared to that before the administration.

Disclosed herein is a method for predicting options of depression treatment drugs, comprising the following steps:
(1) measuring a PEA level in blood collected from a patient suspected of depression;
(2) measuring whether the blood PEA level of the patient is lower than 1.5 µM or not, wherein if the measured value of the patient is lower than 1.5 µM, the patient suffers from depression exhibiting a decreased PEA level as an indicator; and
(3) predicting options of depression treatment drugs on the basis of the presence or absence of decreased blood PEA level of the patient.

In the present specification, the "depression exhibiting a decreased PEA level as an indicator" means a depression involving, as an indicator, the fact that a blood PEA level of a patient was measured in practice and the measured value was detected to be lower than 1.5 µM. Even if a patient potentially has a low blood PEA level, depression of the patient whose PEA level was not measured in practice does not fall under depression exhibiting a decreased PEA level as an indicator.

In the step (3), it is predicted that a serotonin/noradrenaline reuptake inhibitor as a depression treatment drug is preferably selected for e.g. the depressed patient for whom the decreased blood PEA level was observed.

Also, disclosed herein is a method for predicting effects of depression treatment drugs, comprising the following steps:
(1) measuring a PEA level in blood collected from a depressed patient;
(2) measuring whether the blood PEA level of the patient is lower than 1.5 µM or not, wherein if the measured value of the patient is lower than 1.5 µM, the patient suffers from depression exhibiting a decreased PEA level as an indicator; and
(3) predicting that a serotonin/noradrenaline reuptake inhibitor is effective as a depression treatment drug for the depressed patient for whom the decreased blood PEA level was observed.

Also, disclosed herein is a method for predicting a prognosis of treatment with depression treatment drugs, comprising the following steps:
(1) measuring a PEA level in blood collected from a depressed patient before administration of the therapeutic drug;
(2) measuring whether the PEA level of the patient is lower than 1.5 µM or not, wherein if the measured value of the patient is lower than 1.5 µM, the patient suffers from depression exhibiting a decreased PEA level as an indicator;
(3) measuring the PEA level in the blood collected from the depressed patient for whom the decreased blood PEA level was observed in the step (2) and to whom the depression treatment drug was administered; and
(4) evaluating effects caused by the depression treatment drug treatment for the patient on the basis of the measured value of the blood PEA level of the patient to whom a serotonin/noradrenaline reuptake inhibitor was administered as a depression treatment drug.

In the step (3)', the depression treatment drug is preferably a serotonin/noradrenaline reuptake inhibitor.

In the step (4), it is preferable to evaluate the depression treatment drug as effective on the basis of the fact that the blood PEA level of the patient after administration of the therapeutic drug was higher than 1.5 µM.

In the step (4), it is preferable to evaluate the prognosis of the patient as partial depression remission or depression remission on the basis of the increase degree of the blood PEA level of the patient after administration of the therapeutic drug compared to that before the administration.

Also, disclosed herein is a depression treatment drug characterized in that:
a patient for whom the drug is prescribed is a subject whose measured value of the blood PEA level was detected to be lower than 1.5 µM; and
the depression treatment drug comprises a serotonin/noradrenaline reuptake inhibitor. Although the serotonin/noradrenaline reuptake inhibitor (SNRI) is known as a depression treatment drug, the present invention is characterized in that the subject to whom the inhibitor is administered is limited to a subject who was actually confirmed to have a lower measured value of the blood PEA level than 1.5 µM.

### Effects of Invention

According to the method for predicting the options of the depression treatment drugs of the present invention, the options of the depression treatment drugs can be objectively and easily predicted on the basis of the presence or absence of decrease in the blood PEA level. For example, it is preferable to select a serotonin/noradrenaline reuptake inhibitor as a depression treatment drug for a depressed patient for whom a decreased blood PEA level was observed. The prediction can be a basis of diagnosis by a physician who prescribes drugs in practice.

According to the method for predicting the effect of the depression treatment drug of the present invention, it can be predicted that selection of the SNRI as a depression treatment drug is effective for a depressed patient for whom a decreased PEA level was observed. The prediction is useful for diagnosis by a physician who prescribes drugs in practice.

According to the method for predicting the prognosis of the treatment with the depression treatment drug of the present invention, the process of remission after administration of the depression treatment drug can be monitored by the blood PEA level for a depressed patient for whom a decreased blood PEA level was observed. For example, the process of remission after administration of the SNRI to the patient for whom the decreased PEA level was observed can be objectively evaluated by the increase degree of the blood PEA level. This prediction provides useful information with a high added value to physician's diagnosis.

### Description of Embodiments

Hereinafter, one embodiment of the present invention will be described in more detail. A method for predicting options of depression treatment drugs of the present invention comprises the following steps:
(1) measuring a phosphoethanolamine level in blood collected from a patient suspected of depression; and
(2) measuring whether the blood phosphoethanolamine level of the patient is lower than 1.5 µM or not;
wherein if the measured value of the patient is lower than 1.5 µM, the patient suffers from depression exhibiting a decreased phosphoethanolamine level as an indicator and a serotonin/noradrenaline reuptake inhibitor is selected as depression treatment drug for the patient.

In the method of the present invention, a blood PEA level of a patient suspected of depression is measured in practice, and a depression in which a lower measured value than the PEA level in blood collected from healthy persons was detected is subjected to the method. The depression exhibiting a decreased PEA level as an indicator is in an acute phase of depression.

By using a decreased blood PEA level lower than 1.5 µM as an indicator for determining depression, anxiety disorder resemblant to depression can be easily and objectively distinguished from depression. This anxiety disorder includes generalized anxiety disorder, social anxiety disorder, panic disorder, obsessive compulsive disorder, adjustment disorder, hypochondria, dissociative disorder and somatoform disorder.

The blood collected in the step (1) may be blood itself and serum and plasma separated from the blood. Preferably it is serum or plasma. The method for separating serum or plasma from blood is not particularly limited, and it includes a stationary method, a centrifugal method and the like, for example.

The blood PEA level can be measured by a known general-purpose method, which includes, for example, an ion chromatography-fluorescent detection (IC-FLD) method, a high performance liquid chromatography-mass spectrometer (LC-MS) method, a gas chromatography-mass spectrometer (GC-MS) method, a capillary electrophoresis-mass spectrometer (CE-MS) method, an NMR analysis, an acid-alkali neutralization titration, an amino acid analysis, an enzyme method, a method using a nucleic acid aptamer/peptide aptamer or antibody, a colorimetric method, a high performance liquid chromatography, a gas chromatography, a mass spectrometry and the like.

Since the blood PEA is very small amount, the IC-FLD method, the CE-MS method, the LC-MS method, the GC-MS method, the high performance liquid chromatography, the enzyme method and the antibody method, which can provide a high measurement accuracy, are preferable.

The blood PEA level may be either an absolute concentration or a comparable value of the absolute concentration of each individual relative to the absolute concentration. Examples of such values may include a relative concentration, a weight per unit volume, raw data measured for acquiring the absolute concentration (e.g. a value obtained by standardizing a peak area in a graph obtained by measurement using the CE-MS method) and the like. Preferably, it is an absolute concentration.

In the step (2), a blood PEA level of the patient suspected of depression is compared with a reference value of 1.5 µM.

Blood PEA levels of depressed patients and non-depressed patients are shown in Table 1.

**[Table 1]**

| | Disease | n | Blood PEA level Average value ±SD(*µ*M) |
|---|---|---|---|
| Non-depression | Healthy person | 72 | 2.03±0.46 |
| | Depression remission | 53 | 1.98±0.47 |
| | Anxiety disorder (including panic disorder and adjustment disorder) | 349 | 2.04±0.52 |
| Depression | Partial depression remission | 97 | 1.74±0.40 |
| | Depression (acute phase) | 354 | 1.29±0.17 |

On the basis of the results in Table 1, the reference value for detecting the depression exhibiting the decreased PEA level as an indicator in the present invention is 1.5 µM.

In the step (3), on the basis of the presence or absence of the decreased blood PEA level, the options of depression treatment drugs are predicted.

If the blood PEA level of a patient is lower than 1.5 µM, the patient suffers from depression exhibiting the decreased PEA level as an indicator. The inventors found that administering SNRI was more effective than SSRI as a depression treatment drug for such a patient. Therefore, it is preferable to predict an option of the SNRI as a depression treatment drug for the depressed patient for whom the decreased blood PEA level was observed in the step (3).

The SNRI approved in Japan includes duloxetine (trade name: Cymbalta (registered trademark)) and milnacipran (trade name: Toledomin (registered trademark)). In a depressed patient for whom a decreased blood PEA level was observed, the blood PEA level is increased by the SNRI administration.

When a blood PEA level of a patient is not less than 1.5 µM, the disease of the patient does not fall under depressions exhibiting the decreased PEA level as an indicator even if the symptoms are similar to depression. The disease is suspected of anxiety disorder. Indeed, as a drug for treating patients with anxiety disorder, the SSRI having an effect of calming down anxiety and fear is effective. The SSRI approved in Japan includes fluvoxamine (trade name: Deplomel (registered trademark), luvox (registered trademark)), paroxetine (trade name: Paxil (registered trademark)), sertraline (trade name: Jzoloft (registered trademark)) and escitalopram (trade name: Lexapro (registered trademark)).

Also, disclosed herein is a method for predicting a prognosis of treatment with depression treatment drugs, comprising the following steps:
(1) measuring a PEA level in blood collected from a depressed patient before administration of the therapeutic drug;
(2) measuring whether the PEA level of the patient is lower than 1.5 µM or not, wherein if the measured value of the patient is lower than 1.5 µM, the patient suffers from depression exhibiting a decreased PEA level as an indicator;
(3) measuring the PEA level in the blood collected from the depressed patient for whom the decreased blood PEA level was observed in the step (2) and to whom the depression treatment drug was administered; and
(4) evaluating the prognosis of the patient caused by the depression treatment drug treatment on the basis of the measured value of the blood PEA level of the patient to whom a serotonin/noradrenaline reuptake inhibitor was administered as a depression treatment drug.

For the patient suffering from depression exhibiting the decreased PEA level as an indicator whom the decreased blood PEA level was observed in the step (2), a depression treatment drug may be prescribed according to a doctor's diagnosis. As the depression treatment drug, the SNRI is more preferable than the SSRI.

In the step (3), the PEA level in the blood collected from the patient after administration of the depression treatment drug is measured.

The inventors found that a course of the prognosis could be evaluated according to transition of a blood PEA level after administration of the depression treatment drug. The relationship between the course of the prognosis for depression and the blood PEA level is summarized in Table 2.

**[Table 2]**

| | Blood PEA level (*µ*M) |
|---|---|
| Acute phase of depression | 1.29±0.17 |
| Partial depression remission | 1.74±0.40 |
| Depression remission | 1.98±0.47 |

In the step (4), the prognosis by the treatment of the depression treatment drug for the patient is evaluated on the basis of the measured value of the blood PEA level of the patient to whom the depression treatment drug was administered, and Table 2. Depending on individual differences among patients, when the blood PEA level exceeds about 1.8 µM, the patient gets quite good health. This phase corresponds to a partial depression remission. The patient heads towards a termination of the treatment according to a depression treatment guideline. At the termination phase of the treatment, the PEA value is increased to 1.5 to 2.5 µM and stabilized. If the value is stable in a range of 1.5 to 2.5 µM even after the dosage is then decreased, a depression remission is achieved. Thus, the blood PEA level can be used for not only depression diagnosis but also a prognostic criterion of the depression treatment.

Also, disclosed herein is a depression treatment drug characterized in that:
a patient for whom the drug is prescribed is a subject whose measured value of the blood PEA level was detected to be lower than 1.5 µM; and
the depression treatment drug comprises a serotonin/noradrenaline reuptake inhibitor. The depression treatment drug of the present invention is prescribed for a subject whose measured value of a blood phosphoethanolamine level was detected to be lower than that of healthy persons. That is, as for a patient for whom the depression treatment drug of the present invention is prescribed, a patient who potentially has a low blood PEA level but did not actually undergo the PEA level measurement is excluded.

Also, disclosed herein is a method for treatment with depression treatment drugs, comprising the following steps:
measuring a phosphoethanolamine level in blood collected from a depressed patient;
measuring whether the blood phosphoethanolamine level of the patient is lower than 1.5 µM or not, wherein if the measured value of the patient is lower than 1.5 µM, the patient suffers from depression exhibiting a decreased phosphoethanolamine level as an indicator;
determining that a serotonin/noradrenaline reuptake inhibitor is effective as a depression treatment drug for the depressed patient for whom the decreased blood phosphoethanolamine level was observed; and
prescribing the serotonin/noradrenaline reuptake inhibitor to treat the depressed patient.

Also, disclosed herein is a facility for predicting options of depression treatment drugs, comprising:
an apparatus for measuring a phosphoethanolamine level in blood collected from a patient suspected of depression;
an apparatus for measuring whether the level of the patient is lower than 1.5 µM or not, wherein if the level of the patient is lower than 1.5 µM, the patient is determined to suffer from depression exhibiting a decreased phosphoethanolamine level as an indicator, and
an apparatus for outputting a prediction result for the options of the depression treatment drugs on the basis of the presence or absence of the decreased blood phosphoethanolamine level of the patient.

Also, disclosed herein is an application of an apparatus for measuring phosphoethanolamine for manufacturing a facility for predicting options of depression treatment drugs, wherein the facility comprises:
the apparatus for measuring the phosphoethanolamine level in blood collected from a patient suspected of depression;
an apparatus for measuring whether the level of the patient is lower than 1.5 µM or not, wherein if the level of the patient is lower than 1.5 µM, the patient is determined to suffer from depression exhibiting a decreased phosphoethanolamine level as an indicator; and an apparatus for outputting a prediction result for the options of the depression treatment drugs on the basis of the presence or absence of the decreased blood phosphoethanolamine level of the patient.

Also, disclosed herein is an application of phosphoethanolamine for manufacturing a facility for predicting options of depression treatment drugs, wherein the facility comprises:
an apparatus for measuring a phosphoethanolamine level in blood collected from a patient suspected of depression;
an apparatus for measuring whether the level of the patient is lower than 1.5 µM or not, wherein if the level of the patient is lower than 1.5 µM, the patient is determined to suffer from depression exhibiting a decreased phosphoethanolamine level as an indicator; and
an apparatus for outputting a prediction result for the options of the depression treatment drugs on the basis of the presence or absence of the decreased blood phosphoethanolamine level of the patient.

Also, disclosed herein is a computer program for predicting options of depression treatment drugs, which allows a computer to execute:
a step of measuring the phosphoethanolamine level in blood collected from a patient suspected of depression;
a step of measuring whether the level of the patient is lower than 1.5 µM or not, wherein if the level of the patient is lower than 1.5 µM, the patient is determined to suffer from depression exhibiting a decreased phosphoethanolamine level as an indicator; and
a step of outputting a prediction result for the options of the depression treatment drugs on the basis of the presence or absence of the decreased blood phosphoethanolamine level of the patient.

Also, disclosed herein is a computer-readable recording medium on which a computer program for predicting options of depression treatment drugs is recorded.

### Examples

### [Example 1] Measurement of blood PEA level of a depressed patient before and after administration

In order to investigate a correlation between the depression exhibiting the decreased PEA level as an indicator and the depression treatment drug, the following analysis was carried out. After an informed consent was given to a patient who visited the clinic of the inventors, the patient's blood was collected. The blood PEA level was measured using an ion chromatography fluorescent detection method according to the following procedure.

### 1. Pretreatment

50 µL of plasma and 450 µL of Milli-Q water were put in an Eppendorf tube and mixed by vortexing. The mixture was transferred to an ultrafiltration filter (Ultrafree-MC-PLHCC 5 kDa cut filter, Millipore Corporation), and subjected to centrifugal filtration (4°C, 9, 100×g, 2 hours) until the solution in the filter disappeared. The filtered solution (sample derived from plasma) was subjected to measurement by the ion chromatography fluorescent detection method.

### 2. Measuring apparatus for ion chromatography fluorescent detection method

Ion chromatography (product name: ICS-5000, Thermo Fisher Scientific Inc.) Thermoreactor (product name: Nanospace 3019 SI-2, Shiseido Company, Limited) Fluorescence detector (product name: UltiMate 3000, Thermo Fisher Scientific Inc.)

### 3. Measurement condition

Buffer: 20 mM sodium hydroxide solution
Column: strong anion exchange column (product name: IonPac AS20, Thermo Fisher Scientific Inc.)
Column temperature: 35°C
Fluorescence reagent: orthophthalaldehyde
Derivatization method: post-column derivatization
Reaction temperature: 35°C
Excitation wavelength: 340 nm
Fluorescence wavelength: 450 nm

### 4. Calculation of concentration

A PEA level in the plasma was calculated using a calibration curve derived from a peak area of the PEA of the plasma-derived sample and a peak area of the PEA standard solution.

As subjects, 30 patients having the blood PEA levels ranging from 0.77 to 2.62 µM were employed.

As a depression treatment drug, the SSRI or SNRI shown in Table 3 was administered to the patients. The blood PEA level after the administration was measured in the same procedure as before the administration. Although the administration periods variably depended on the patients, they ranged from about 0.5 to 7 months. The results are shown in Table 3.

**[Table 3]**

| Name of drug | | Blood PEA level (*µ*M) | | t-test | |
|---|---|---|---|---|---|
| | | Before administration | After administration | p-value | Significant difference (level of significance of 5%) |
| SNRI (n=18) | Duloxetine(n=10) | 1.10 | 1.46 | 0.02 | Present |
| | Milnacipran(n=8) | 1.27 | 1.66 | 0.07 | Present |
| | Whole | 1.18 | 1.55 | 0.002 | Present |
| SSRI (n=12) | Escitalopram(n=5) | 1.21 | 1.22 | 0.88 | Absent |
| | Sertraline(n=5) | 1.39 | 1.26 | 0.39 | Absent |
| | Paroxetine(n=2) | 1.42 | 1.21 | - | - |
| | Whole | 1.34 | 1.23 | 0.39 | Absent |

The blood PEA level before the administration of whole patients to whom the SNRI was administered was 1.18 µM (average value). After the administration, the blood PEA level was increased to 1.55 µM. A p-value in the t-test was 0.002, in which a significant difference was recognized at the level of significance of 5%. A patient at an acute phase of depression before administrating the SNRI was recognized to progress to a partial remission phase of depression after the administration period.

On the other hand, the blood PEA level before the administration of whole patients to whom the SSRI was administered was 1.34 µM (average value). After the administration, the blood PEA level was 1.23 µM. Although the blood PEA level was slightly decreased by administering the SSRI, a p-value in the t-test was 0.39, in which no significant difference was recognized at the level of significance of 5%. A patient at an acute phase of depression before administering the SSRI was recognized to remain in the acute phase of depression even after the administration period.

The above results show that when a kind of antidepressants, SNRI is administered to a depressed patient exhibiting a decreased blood PEA level as an indicator, particularly a depressed patient exhibiting a blood PEA level before the administration of not more than 1.5 µM, the blood PEA level is evidently increased. The SNRI is effective in that it rises the blood PEA level of a depressed patient exhibiting a decreased blood PEA level as an indicator. On the other hand, even if a kind of antidepressants, SSRI is administered to a depressed patient exhibiting a decreased blood PEA level as an indicator, particularly a depressed patient exhibiting a pre- blood PEA level before the administration of not more than 1.5 µM, the blood PEA level is not increased. Even if the SSRI calms down anxiety and fear of the depressed patient, it is ineffective on increasing the blood PEA level of the patient. It is suggested that the administration of the SNRI is more effective than of the SSRI for treating a depression exhibiting a decreased blood PEA level as an indicator.

### [Example 2] Measurement of blood PEA level of depressed patient during continuous administration

Among the patients to whom the depression treatment drug was administered in Example 1, the patients who received continuous administration also after the period of Example 1 were investigated. The number of patients who received continuous administration of the SNRI was nine. On the other hand, the number of patients who received continuous administration of the SSRI was five. Although the continuous administration periods (including the administration period in Example 1) variably depended on the patients, they ranged from about 6 to 24 months. The blood PEA levels in the course of and after the end of the continuous administration of these patients were measured in the same procedure as in Example 1. The results are shown in Table 4.

**[Table 4]**

| Name of drug | | Blood PEA level (*µ*M) | | | t-test 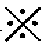 | |
|---|---|---|---|---|---|---|
| | | Before administration 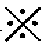 | During continuous administration | After continuous administration | p-value | Significant difference (level of significance of 5%) |
| SNRI (n=9) | Duloxetine (n=7) | 1.11 | 1.47 | 1.82 | 0.02 | Present |
| | Milnacipran (n=2) | 1.44 | 1.71 | 1.94 | - | - |
| | Whole | 1.28 | 1.53 | 1.88 | 0.0005 | Present |
| SSRI (n=5) | Escitalopram(n=1) | 1.20 | 1.21 | 1.73 | - | - |
| | Sertraline(n=4) | 1.39 | 1.24 | 1.47 | 0.68 | Absent |
| | Whole | 1.30 | 1.23 | 1.60 | 0.19 | Absent |

| | | | | | | |
|---|---|---|---|---|---|---|
| Before administration: Before administration in Example 1 t-test: Significant difference test of blood PEA level after continuous administration with respect to that before administration | | | | | | |

The PEA level before the administration of the whole patients to whom the SSRI was administered was 1.28 µM (average value). The PEA level had been consistently increased since the continuous administration and reached 1.88 µM after the continuous administration. A p-value in the t-test was 0.0005, in which a significant difference was recognized at the level of significance of 5%. A patient at an acute phase of depression before administering the SNRI was recognized to have progressed to a remission phase of depression after the administration period.

On the other hand, the average PEA level before the administration of the whole patients to whom the SSRI was administered was 1.30 µM (average value), and the average PEA level after the administration was increased to 1.60 µM. However, it had not been consistently increased since the continuous administration. In addition, some patients exhibited blood PEA levels decreased during the continuous administration, and considerable unevenness was found among the patients. Also, the p-value in the t-test was 0.19, in which no significant difference was recognized at the level of significance of 5%.

The above results show that when a kind of antidepressants, SNRI is continuously administered to a depressed patient exhibiting a decreased blood PEA level as an indicator, particularly a depressed patient exhibiting a blood PEA level before the administration of not more than 1.5 µM, the blood PEA level is evidently increased. The SNRI is effective in that it rises the blood PEA level of a depressed patient exhibiting a decreased blood PEA level as an indicator.

On the other hand, when a kind of antidepressants, SSRI was continuously administered to a depressed patient exhibiting a decreased blood PEA level as an indicator, particularly a depressed patient exhibiting a blood PEA level before the administration of not more than 1.5 µM, the blood PEA level was increased after the continuous administration, but no significant difference was found. Even if the SSRI calms down anxiety and fear of the depressed patient, it is ineffective on increasing the blood PEA level of the patient.

### [Example 3] Measurement of blood PEA level of depressed patient with combined administration of the SNRI and SSRI

The patients to whom a depression treatment drug combining the SNRI and SSRI was administered were investigated. The number of patients who receive administration of the SNRI+SNRI was three. Subsequently, the number of patients who receive administration of the SNRI+SSRI was four. The number of patients who receive administration of the SSRI+SSRI was one. Although the administration periods variably depended on the patients, they ranged from about 2 to 8 months. The blood PEA levels before and after the administration of these patients were measured in the same procedure as in Example 1. The results are shown in Table 5.

**[Table 5]**

| Name of drug | | Blood PEA level (*µ* M) | | t-test | |
|---|---|---|---|---|---|
| | | Before administration | After administration | p-value | Significant difference (level of significance of 5%) |
| SNRI+SNRI (n=3) | Duloxetine + Milnacipran | 1.20 | 1.44 | 0.22 | Absent |
| SNRI+SSRI (n=4) | Duloxetine or Milnacipran + Sertraline or Paroxetine | 1.54 | 1.44 | 0.8 | Absent |
| SSRI+SSRI (n=1) | Escitalopram + Paroxetine | 2.05 | 1.46 | - | - |

The average blood PEA level before the administration of whole patients dosed to whom the SNRI+SNRI was administered was 1.20 µM (average value). After administration, the blood PEA level was increased to 1.44 µM. On the other hand, the blood PEA level before the administration of whole patients to whom the SNRI+SSRI was administered was 1.54 µM, while the blood PEA level after the administration was decreased to 1.44 µM. Additionally, the blood PEA level before the administration of patients to whom the SSRI+SSRI was administered was 2.05 µM (average value), while the blood PEA level after the administration was evidently decreased to 1.46 µM. The above results show that the blood PEA level is evidently increased by changing the combined administration from the SSRI+SSRI to the SNRI+SSRI and further to the SNRI+SNRI.

In Examples 1 to 3, the blood PEA level was significantly increased by administering the SNRI rather than the SSRI to the depressed patients having the blood PEA level of not more than 1.5 µM. It became clear that the administration of the SNRI was effective than that of the SSRI for treating a depression patient exhibiting a decreased blood PEA level as an indicator, particularly a depressed patient exhibiting a blood PEA level before the administration of not more than 1.5 µM.

## Claims

1. A method for predicting options of depression treatment drugs, comprising the following steps:
(1) measuring a phosphoethanolamine level in blood collected from a patient suspected of depression; and
(2) measuring whether the blood phosphoethanolamine level of the patient is lower than 1.5 µM or not;
wherein if the measured value of the patient is lower than 1.5 µM, the patient suffers from depression exhibiting a decreased phosphoethanolamine level as an indicator and a serotonin/noradrenaline reuptake inhibitor is selected as depression treatment drug for the patient.

2. A method for predicting effects of depression treatment drugs, comprising the following steps:
(1) measuring a phosphoethanolamine level in blood collected from a depressed patient; and
(2) measuring whether the blood phosphoethanolamine level in the patient is lower than 1.5 µM or not;
wherein if the measured value of the patient is lower than 1.5 µM, the patient suffers from depression exhibiting a decreased phosphoethanolamine level as an indicator and a serotonin/noradrenaline reuptake inhibitor is predicted to be effective as a depression treatment drug.

3. A method for evaluating a prognosis of treatment with a depression treatment drug which is a serotonin/noradrenaline reuptake inhibitor, comprising the following steps:
(1) measuring a phosphoethanolamine level in blood collected from a depressed patient before administration of the therapeutic drug;
(2) measuring whether the phosphoethanolamine level of the patient is lower than 1.5 µM or not, wherein if the measured value of the patient is lower than 1.5 µM, the patient suffers from depression exhibiting a decreased phosphoethanolamine level as an indicator;
(3) measuring the phosphoethanolamine level in the blood collected from the depressed patient for whom the decreased blood phosphoethanolamine level was observed in the step (2) and to whom the depression treatment drug was administered; and
(4) evaluating the serotonin/noradrenaline reuptake inhibitor as effective on the basis of the fact that the blood phosphoethanolamine level in the patient after administration of the therapeutic drug was higher than 1.5 µM.

4. The method according to claim 3, wherein in the step (4), the prognosis of the patient is evaluated as partial depression remission or depression remission on the basis of the increase degree of the blood phosphoethanolamine level in the patient after administration of the therapeutic drug compared to that before the administration.

## Patentansprüche

1. Verfahren zum Vorhersagen von Optionen von Arzneimitteln zur Behandlung von Depression, umfassend die folgenden Schritte:
(1) Messen eines Phosphoethanolaminspiegels im Blut, das von einem Patienten mit Verdacht auf Depression entnommen wurde; und
(2) Messen, ob der Blut-Phosphoethanolaminspiegel des Patienten geringer als 1,5 µM ist oder nicht;
wobei, wenn der gemessene Wert des Patienten geringer als 1,5 µM ist, der Patient an einer Depression leidet, die einen verringerten Phosphoethanolaminspiegel als einen Indikator anzeigt und für den Patienten ein Serotonin-/Noradrenalin-Wiederaufnahmehemmer ausgewählt wird als Arzneimittel zur Behandlung von Depressionen.

2. Verfahren zum Vorhersagen der Wirkungen von Arzneimitteln zur Behandlung von Depressionen, umfassend die folgenden Schritte:
(1) Messen eines Phosphoethanolaminspiegels im Blut, das von einem depressiven Patienten entnommen wurde; und
(2) Messen, ob der Blut-Phosphoethanolaminspiegel im Patienten geringer als 1,5 µM ist oder nicht;
wobei, wenn der gemessene Wert des Patienten geringer als 1,5 µM ist, der Patient an einer Depression leidet, die einen verringerten Phosphoethanolaminspiegel als einen Indikator anzeigt und ein Serotonin-/Noradrenalin-Wiederaufnahmehemmer voraussichtlich als ein Arzneimittel zur Behandlung von Depressionen wirksam ist.

3. Verfahren zum Auswerten einer Prognose der Behandlung mit einem Arzneimittel zur Behandlung von Depressionen, das ein Serotonin-/Noradrenalin-Wiederaufnahmehemmer ist, umfassend die folgenden Schritte:
(1) Messen eines Phosphoethanolaminspiegels im Blut, das von einem depressiven Patienten vor der Verabreichung des therapeutischen Arzneimittels entnommen wurde;
(2) Messen, ob der Phosphoethanolaminspiegel des Patienten geringer als 1,5 µM ist oder nicht, wobei, wenn der gemessene Wert des Patienten geringer als 1,5 µM ist, der Patient an einer Depression leidet, die einen verringerten Phosphoethanolaminspiegel als einen Indikator aufzeigt;
(3) Messen des Phosphoethanolaminspiegels im Blut, das dem depressiven Patienten entnommen wurde, bei dem in Schritt (2) der verringerte Blut-Phosphoethanolaminspiegel beobachtet wurde und dem das Arzneimittel zur Behandlung von Depressionen verabreicht wurde; und
(4) Auswerten des Serotonin/Noradrenalin-Wiederaufnahmehemmers als wirksam auf der Grundlage der Tatsache, dass der Blut-Phosphoethanolaminspiegel des Patienten nach Verabreichung des therapeutischen Arzneimittels höher als 1,5 µM war.

4. Verfahren nach Anspruch 3, wobei in Schritt (4) die Prognose des Patienten als partielle Depressionsremission oder Depressionsremission auf der Grundlage des Anstiegsgrads des Blut-Phosphoethanolaminspiegels des Patienten nach Verabreichung des therapeutischen Arzneimittels im Vergleich zu dem vor Verabreichung ausgewertet wird.

## Revendications

1. Procédé de prédiction d'options de médicaments de traitement de la dépression, comprenant les étapes suivantes :
(1) mesurer un taux de phosphoéthanolamine dans le sang prélevé chez un patient suspecté de dépression ; et
(2) mesurer si le taux de phosphoéthanolamine dans le sang du patient est inférieur à 1,5 µM ou non ;
dans lequel si la valeur mesurée du patient est inférieure à 1,5 µM, le patient souffre de dépression présentant une diminution du taux de phosphoéthanolamine comme indicateur et un inhibiteur de recapture de la sérotonine/noradrénaline est sélectionné comme médicament de traitement de la dépression pour le patient.

2. Procédé de prédiction d'effets de médicaments de traitement de la dépression, comprenant les étapes suivantes :
(1) mesurer un taux de phosphoéthanolamine dans le sang prélevé chez un patient souffrant de dépression ; et
(2) mesurer si le taux de phosphoéthanolamine dans le sang du patient est inférieur à 1,5 µM ou non ;
dans lequel si la valeur mesurée du patient est inférieure à 1,5 µM, le patient souffre de dépression présentant une diminution du taux de phosphoéthanolamine comme indicateur et un inhibiteur de recapture de la sérotonine/noradrénaline est prévu pour être efficace comme médicament de traitement de la dépression.

3. Procédé d'évaluation d'un pronostic de traitement avec un médicament de traitement de la dépression qui est un inhibiteur de recapture de la sérotonine/noradrénaline, comprenant les étapes suivantes :
(1) mesurer un taux de phosphoéthanolamine dans le sang prélevé chez un patient souffrant de dépression avant l'administration du médicament thérapeutique ;
(2) mesurer si le taux de phosphoéthanolamine du patient est inférieur à 1,5 µM ou non, dans lequel si la valeur mesurée du patient est inférieure à 1,5 µM, le patient souffre de dépression présentant une diminution du taux de phosphoéthanolamine comme indicateur ;
(3) mesurer le taux de phosphoéthanolamine dans le sang prélevé chez le patient souffrant de dépression pour lequel la diminution du taux de phosphoéthanolamine dans le sang a été observée à l'étape (2) et auquel le médicament de traitement de la dépression a été administré ; et
(4) évaluer l'inhibiteur de recapture de la sérotonine/noradrénaline comme efficace compte tenu du fait que le taux de phosphoéthanolamine dans le sang du patient après administration du médicament thérapeutique était supérieur à 1,5 µM.

4. Procédé selon la revendication 3, dans lequel à l'étape (4), le pronostic du patient est évalué comme une rémission partielle de la dépression ou une rémission sur la base du degré d'augmentation du taux de phosphoéthanolamine dans le sang du patient après administration du médicament thérapeutique par rapport à celui avant l'administration.
